# FASCICULE DE BREVET EUROPEEN

(11) **EP 0 673 422 B1**
(45) Date de publication et mention de la délivrance du brevet: **04.01.2006**
(21) Numéro de dépôt: 94901994.7
(22) Date de dépôt: 07.12.1993
(51) Int. Cl.: C12N 15/52, C12N 15/68, C12N 15/74, C12P 19/42, C12N 1/21, C12R 1/38

(54) **CELLULES MODIFIEES AU NIVEAU DU CATABOLISME DE LA BETAINE, PREPARATION ET UTILISATIONS, NOTAMMENT POUR LA PRODUCTION DE METABOLITES OU D'ENZYMES**
MODIFIZIERTE ZELLEN AUF DER BASIS DES BETAIN-KATABOLISMUS, HERSTELLUNG UND VERWENDUNG INSBESONDERE ZUR HERSTELLUNG VON METABOLITEN ODER ENZYMEN
CELLS WITH ALTERED BETAINE CATABOLISM, THEIR PREPARATION AND THEIR USE, IN PARTICULAR FOR PRODUCING METABOLITES OR ENZYMES

(30) Priorité: 09.12.1992 FR 9214814
(43) Date de publication de la demande: 27.09.1995
(73) Titulaire: RHONE-POULENC BIOCHIMIE, 92160 Antony Cedex (FR)
(72) Inventeur: CAMERON, Béatrice, F-75005 Paris (FR); CROUZET, Joel, F-75012 Paris (FR)
(74) Mandataire: Becker, Philippe
(86) Numéro de dépôt international: PCT/FR1993/001202
(87) Numéro de publication internationale: WO 1994/013813

(56) Documents cités:
- EP-A- 0 543 344
- DATABASE WPI Week 9134, Derwent Publications Ltd., London, GB; AN 91-252650 & WO,A,91 11518 (RHONE POULENC BIOCHIMIE) 8 Aoüt 1991
- JOURNAL OF BACTERIOLOGY vol. 173, no. 19 , Octobre 1991 , AM. SOC. MICROBIOL., BALTIMORE, US; pages 6058 - 6065 B. CAMERON ET AL. 'Genetic and sequence analyses of a Pseudomonas denitrificans DNA fragment containing two cob genes' cité dans la demande
- JOURNAL OF BACTERIOLOGY vol. 171, no. 1 , Janvier 1989 , AM. SOC. MICROBIOL., BALTIMORE, US; pages 547 - 557 B. CAMERON ET AL. 'Cloning and analysis of genes involved in Coenzyme B12 biosynthesis in Pseudomonas denitrificans' cité dans la demande
- DATABASE WPI Week 8104, Derwent Publications Ltd., London, GB; AN 81-04653D & JP,A,55 148 084 (MITSUBISHI GAS CHEM IND) 19 Novembre 1990
- DATABASE WPI Week 8737, Derwent Publications Ltd., London, GB; AN 87-261317 & JP,A,62 181 791 (AJINIMOTO KK) 10 Aoüt 1987

## Description

La présente invention concerne des cellules modifiées au niveau du catabolisme de la bétaïne, leur préparation et leur utilisation, notamment pour la production améliorée de métabolites et/ou d'enzymes. L'invention concerne également des fragments d'ADN portant des gènes du catabolisme de la bétaïne.

La glycine bétaïne (N,N,N-triméthylglycine) est généralement connue pour ses propriétés osmoprotectrices, conférant aux bactéries une tolérance au stress osmotique (Csonka, 1989). Pour expliquer l'origine de cette propriété, il a été proposé que les effets moléculaires de la glycine bétaïne sur l'activité de l'eau et sur la pression osmotique du cytoplasme chez Escherichia coli étaient plus importants que ceux des solutés qu'elle remplace (Cayley et al, 1992). De plus, en dehors de ses potentialités osmoprotectrices, il a été décrit que la glycine bétaïne pouvait également favoriser la production d'enzymes (JP 8260709) ou de métabolites, tels que des acides aminés (brevet JP 202703) ; des antibiotiques (brevet AU 825513) et des vitamines (White et al, 1971). Toutefois, la plupart des bactéries sauf les cyanobactéries et d'autres procaryotes fixant le CO₂, ne synthétisent pas la glycine bétaïne, qui est principalement synthétisée par les plantes. Celle-ci doit donc être ajoutée aux milieux de production dans les fermenteurs, ce qui génère un coût supplémentaire dans un procédé industriel. La présente invention apporte une solution à ce problème.

La demanderesse a en effet démontré qu'il est possible, en modifiant le catabolisme de la bétaïne des cellules, notamment par des moyens génétiques, de potentialiser l'effet de ce composé sur la production d'enzymes ou de métabolites sans affecter la vitesse de croissance des cellules, leur viabilité, etc, dans des conditions industrielles de fermentation. La demanderesse a également identifié, isolé et caractérisé des fragments d'ADN contenant des gènes impliqués dans le catabolisme de la bétaïne, qui permettent notammant de préparer des cellules spécifiquement modifiées au niveau du catabolisme de la bétaïne, et dont les modifications sont stables ségrégationnellement et non réversibles. Ces fragments permettent également de stimuler le catabolisme de la bétaïne par amplification des activités enzymatiques appropriées. La présente invention permet donc de potentialiser les effets de la bétaïne et, de ce fait, d'utiliser ce composé de manière économique dans des procédés industriels de fermentation.

Un premier objet de l'invention concerne donc une cellule modifiée, présentant une modification au moins au niveau d'un gène impliqué dans le catabolisme de la bétaïne.

Dans un premier mode particulier de réalisation, le terme cellule modifiée désigne plus particulièrement toute cellule présentant une substitution et/ou une délétion et/ou une insertion d'une ou plusieurs bases dans le ou les gènes considérés et dégradant moins rapidement la bétaïne. De telles modifications peuvent être obtenues in vitro (sur les fragments d'ADN isolés portant des gènes du catabolisme de la bétaïne) ou in situ, par exemple, au moyen des techniques du génie génétique, ou encore en exposant lesdites cellules à un traitement au moyen d'agents mutagènes.

Par agents mutagènes, on peut citer par exemple les agents physiques tels que les rayonnements énergétiques (rayons X, g, ultra violet, etc..), ou les agents chimiques capables de réagir avec différents groupements fonctionnels des bases de l'ADN, et par exemple les agents alkylants [éthylméthane sulfonate (EMS), N-méthyl-N'-nitro-N-nitrosoguanidine, N-nitroquinoléine-1-oxyde (NQO)], les agents bialkylants, les agents intercalants, etc...

Par délétion on entend la suppression de tout ou partie du gène considéré. Il peut s'agir notamment d'une partie de la région codante et/ou de tout ou partie de la région promotrice de la transcription.

Les modifications génétiques peuvent également être obtenues par disruption génique, par exemple selon le protocole initialement décrit par Rothstein (1983). Dans ce cas, tout ou partie du gène est préférentiellement perturbé pour permettre le remplacement, par recombinaison homologue, de la séquence génomique sauvage par une séquence non fonctionnelle ou mutante préparée in vitro.

La ou lesdites modifications peuvent être localisées dans la partie codante du gène ou dans les régions responsables de l'expression et/ou de la régulation transcriptionelle desdits gènes. L'incapacité (totale ou partielle) desdites cellules à dégrader la bétaïne peut se manifester soit par la production d'enzymes inactives en raison de modifications structurales ou conformationelles, soit par l'absence de production, soit par la production d'enzymes ayant une activité altérée, ou encore par la production d'enzymes naturelles à un niveau atténué ou selon un mode de régulation désiré.

Par ailleurs, certaines modifications telles que des mutations ponctuelles sont par nature capables d'être corrigées ou atténuées par des mécanismes cellulaires, par exemple lors de la réplication de l'ADN précédant la division cellulaire. De telles modifications génétiques ont alors un intérêt limité au niveau industriel puisque les propriétés phénotypiques qui en résultent ne sont pas parfaitement stables. Selon la présente invention, il est maintenant possible grâce à l'identification de fragments d'ADN portant des gènes du catabolisme de la bétaïne, de préparer des cellules modifiées dans lesquelles la ou lesdites modifications sont stables ségrégationellement et/ou non-réversibles. Les cellules présentant de telles modifications sont particulièrement avantageuses comme hôte cellulaire pour la production de métabolites et/ou d'enzymes.

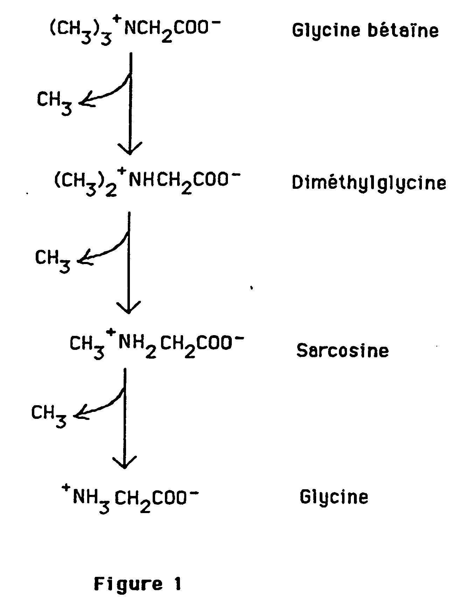

D'après les études, effectuées essentiellement chez Rizobium meliloti ; la dégradation de la glycine bétaïne se réalise sur des milieux de faible osmolarité, par trois déméthylations successives (voir figure 1). La première étape est catalysée par la bétaïne-homocystéine méthyltransférase E.C. 2.1.1.5. et conduit à la diméthylglycine ; la deuxième est catalysée par la diméthylglycine déshydrogénase E.C. 1.5.99.2. et génère la monométhylglycine ou sarcosine ; enfin la troisième est catalysée par la sarcosine déshydrogénase E.C. 1.5.99.1 et le produit de la réaction est la glycine (Smith et al, 1988).

Préférentiellement, les modifications que présentent les cellules de l'invention affectent l'une des deux premières étapes du catabolisme de la bétaïne, ou éventuellement les deux premières étapes simultanément.

Préférentiellement, les cellules de l'invention sont des cellules productrices de métabolites et/ou d'enzymes. A cet égard, il peut également s'agir de cellules recombinées productrices de métabolites et/ou d'enzymes, c'est-à-dire de cellules modifiées par les techniques de l'ADN recombinant en vue d'améliorer leur capacité de production (Cf. notamment WO 91/11518, EP 346000). Encore plus préférentiellement, les cellules de l'invention sont choisies parmi les cellules du genre Pseudomonas, Streptomyces, actinomycètes, Propionibacterium, Corynebacterium, Bacillus, Escherichia, Salmonella, Rhizobium, Agrobacterium, Rhodopseudomonas, Xanthomonas, Clostridium et Methanobacterium.

Un autre aspect de l'invention concerne un procédé de préparation de cellules présentant une modification d'un gène au moins impliqué dans le catabolisme de la bétaïne, utilisables dans des conditions industrielles de fermentation.

La présente invention décrit en effet l'identification, l'isolement et la caractérisation de fragments d'ADN contenant des gènes impliqués dans le catabolisme de la bétaïne. Ces fragments permettent maintenant de préparer des cellules spécifiquement modifiées au niveau du catabolisme de la bétaïne. Il est en effet possible de modifier in vitro les fragments décrits pour les rendre non fonctionnels et de les réintroduire dans une cellule déterminée, dans laquelle ils vont se substituer par double recombinaison homologue à la copie génomique fonctionnelle correspondante. Il est également possible, sur la base des fragments ainsi isolés, d'élaborer des sondes qui s'intègreront dans le génome d'une cellule désirée, spécifiquement dans le gène correspondant.

Plus particulièrement, le procédé de l'invention consiste à remplacer le ou les gènes chromosomiques considérés par des versions modifiées in vitro.

Un autre objet de l'invention concerne un fragment d'ADN portant un gène au moins du catabolisme de la bétaïne.

Plus particulièrement, la présente invention est illustrée par l'isolement et la caractérisation de fragments d'ADN de Pseudomonas denitrificans complémentant les mutants bloqués dans le catabolisme de la glycine bétaïne en diméthylglycine et ceux bloqués dans le catabolisme de la diméthylglycine en samosine ; c'est-à-dire les fragments d'ADN contenant des gènes impliqués dans la dégradation de la glycine bétaïne en diméthylglycine et de la diméthylglycine en sarcosine. Les fragments d'ADN selon la présente invention ont été isolés à partir d'une souche de Pseudomonas denitrificans SC510, dérivée de la souche MB580 (brevet US 3 018 225). Ces fragments ont été obtenus par :
(i) préparation de mutants bloqués dans le catabolisme de la bétaïne. Différentes techniques déjà mentionnées plus haut sont utilisables à cet effet. La sélection des mutants se fait par culture sur milieu approprié et dosage, selon les techniques classiques pour l'homme du métier, de la bétaïne ou des produits de son catabolisme.
(ii) complémentation de ces mutants avec l'acide nucléique d'un microorganisme capable de cataboliser la bétaïne,
(iii) sélection des mutants complémentés, puis isolement et caractérisation de l'acide nucléique ayant permis cette complémentation, qui porte donc des gènes du catabolisme de la bétaïne.

D est clair que, à partir des fragments d'ADN identifiés et isolés dans la présente demande, l'homme du métier peut, notamment par des expériences d'hybridation, isoler et cloner des gènes du catabolisme de la bétaïne à partir d'autres sources cellulaires.

Plus préférentiellement, il s'agit donc du gène codant pour la bétaïne-homocystéine méthyltransférase, sur lequel une modification selon l'invention induit dans la cellule une diminution de l'activité bétaïne-homocystéine méthyltransférase. Toujours préférentiellement, il s'agit du gène codant pour la diméthylglycine déshydrogénase, sur lequel, une modification selon l'invention induit dans la cellule une diminution de l'activité diméthylglycine déshydrogénase.

Un autre objet de l'invention concerne un procédé amélioré de production métabolites ou d'enzymes par culture d'une cellule productrice dudit métabolite ou enzyme, modifiée au niveau de son catabolisme de la bétaïne, dans des conditions de production, puis récupération dudit métabolite ou enzyme.

Selon un premier mode de réalisation, la cellule productrice présente une modification au moins au niveau d'un gène impliqué dans le catabolisme de la bétaïne et dégrade moins rapidement la bétaïne.

Préférentiellement, selon le premier mode de réalisation, la ou les modifications sont stables ségrégationnellement et/ou non réversibles.

Dans une variante préférée de l'invention, la ou les modifications sont des délétions et/ou des insertions mutationnelles.

Selon un deuxième mode de réalisation, un gène au moins de la cellule productrice, impliqué dans le catabolisme de la bétaïne, est amplifié et ladite cellule dégrade plus rapidement la bétaïne.

Le procédé de l'invention est particulièrement adapté à la production de métabolites tels que les acides aminés, les vitamines, les antibiotiques, leurs dérivés ou leurs précurseurs.

Le procédé de l'invention peut notamment permettre la production améliorée de cobalamine, et de préférence de vitamine B12.

La présente invention est complétée par les exemples qui suivent, qui doivent être considérés comme illustratifs et non limitatifs.

### TECHNIQUES GENERALES DE BIOLOGIE MOLECULAIRE

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans *Escherichia coli* etc, sont bien connues de l'homme de métier et sont abondamment décrites dans la littérature [Maniatis T. et al., "Molecular Cloning, a Laboratory Manual", Cold Spring Harbor Laboratory, Cold Spring Harbor, N.Y., 1982 ; Ausubel F.M. et al. (eds), "Current Protocols in Molecular Biology", John Wiley & Sons, New York, 1987].

Les enzymes de restriction ont été fournies par New England Biolabs (Biolabs), ou Pharmacia et sont utilisées selon les recommandations des fournisseurs.

Les plasmides de type pBR322 et pUC sont d'origine commerciale (Bethesda Research Laboratories).

Pour les ligatures, les fragments d'ADN sont séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Boehringer) selon les recommandations du fournisseur.

Le remplissage des extrémités 5' proéminentes est effectué par le fragment de Klenow de l'ADN Polymérase I d'*E.coli* selon les spécifications du fournisseur. La destruction des extrémités 3' proéminentes est effectuée en présence de l'ADN Polymérase du phage T4 utilisée selon les recommandations du fabricant. La destruction des extrémités 5' proéminentes est effectuée par un traitement ménagé par la nucléase S1.

La mutagénèse dirigée *in vitro* par oligodéoxynucléotides synthétiques est effectuée selon la méthode développée par Taylor et al. [Nucleic Acids Res. 13 (1985) 8749-8764].

L'amplification enzymatique de fragments d'ADN par la technique dite de PCR [Polymérase-catalyzed Chain Reaction, Saiki R.K. et al., Science 230 (1985) 1350-1354 ; Mullis K.B. et Faloona F.A., Meth. Enzym. 155 (1987) 335-350] est effectuée en utilisant un "DNA thermal cycler" (Perkin Elmer Cetus) selon les spécifications du fabricant

La vérification des séquences nucléotidiques est effectuée par la méthode développée par Sanger et al. [Proc. Natl. Acad. Sci. USA, 74 (1977) 5463-5467].

### ABREVIATIONS

Bétaïne : glycine bétaïne ou N, N, N-triméthylglycine
CLHP : chromatographie liquide haute pression
DMG : diméthylglycine
kb : kilobase

### LEGENDE DES FIGURES

Figure 1 : Voie de dégradation de la glycine bétaïne en glycine. Trois déméthylations successives ont lieu, la glycine bétaïne est catabolisée en diméthylglycine qui est dégradée en sarcosine elle-même dégradée en glycine.

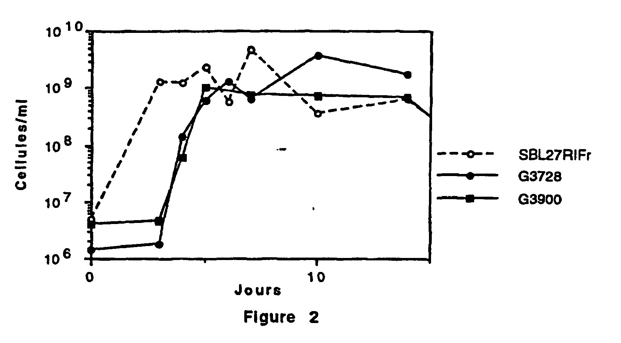

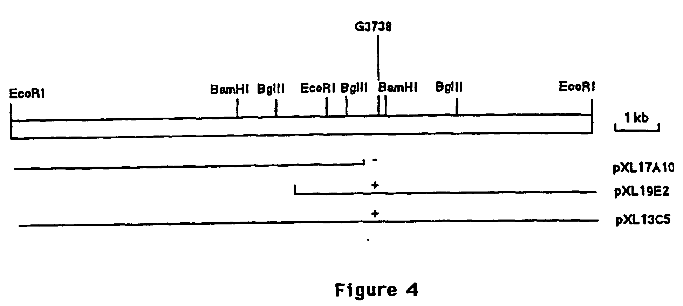

Figure 2 : Courbe de croissance en milieu minimum M. La souche non mutée SBL27 Rif^{r} et les mutants G3728 et G3900 sont cultivés en milieu minimum M en présence de glycine bétaïne comme source de carbone, leur croissance est représentée sur l'axe des ordonnées en nombre de cellules par ml, en fonction du temps en jours décrit sur l'axe des abscisses.

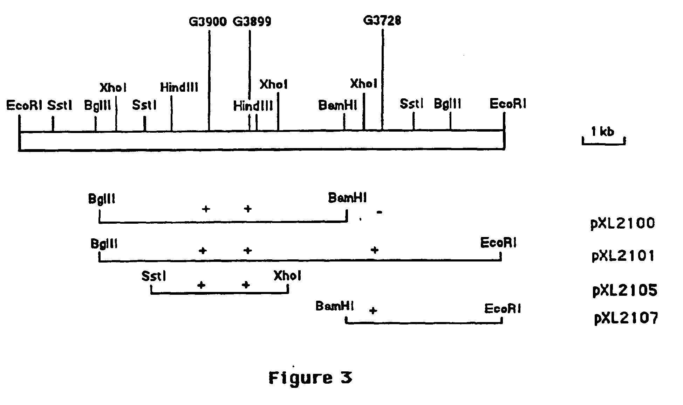

Figure 3 : Cartographie du fragment d'ADN de P. denitrificans complémentant les mutants G3728, G3736, G3899 et G3900. Sur le fragment d'ADN sont indiqués les sites de restriction et la position de l'insertion du transposon des mutants correspondant G3728, G3899 et G3900. Au dessous du fragment sont représentés les inserts des plasmides pXL2100, pXL2101, pXL2105 et pXL2107 qui ont été utilisés pour complémenter les mutants. + complémentation, - pas de complémentation.
Figure 4 : Cartographie du fragment d'ADN de P. denitrificans complémentant les mutants G3727 et G3738. Sur le fragment d'ADN sont indiqués les sites de restriction et la position de l'insertion du transposon de G3738. Au dessous du fragment figure une partie des inserts des plasmides pXL19E2, pXL17A10, et pXL13C5 qui ont été utilisés pour complémenter les mutants. + complémentation, - pas de complémentation.

### EXEMPLE 1- Isolement de mutants de Pseudomonas denitrificans bloqués dans le catabolisme de la glycine bétaïne.

Cet exemple décrit l'isolement de mutants de Pseudomonas denitrificans bloqués dans le catabolisme de la glycine bétaïne en diméthylglycine et sarcosine. Ces mutants ont été isolés à partir d'une banque de mutants dans laquelle le transposon Tn5Sp^{r} est inséré dans le génome de la souche Pseudomonas denitrificans SBL27 Rif^{r}. Cette banque a été réalisée de la façon suivante : Un plasmide, désigné pRK2013::Tn5Sp^{r}, a été construit en insérant le gène de résistance à la spectinomycine Sp^{r} issu du plasmide pHP45Ω (Prentki et al., 1984) au site BamHI du transposon Tn5 (Berg et al., 1983) cloné dans le plasmide pRK2013::Tn5 (Ditta et al., 1980). Une conjugaison a ensuite été réalisée en mélangeant les cultures en phase exponentielle de SBL27Rif^{r} et E.coli MC1060 (pRK2013::Tn5Sp^{r}). Les transconjugants Rif^{r}Sp^{r} ont été obtenus après incubation à 30°C pendant 5 jours, à la fréquence de 10⁻⁸ clones par cellule réceptrice. D a été vérifié pour 12 clones que le plasmide introduit avait bien été perdu (l'ADN plasmidique a été préparé puis introduit dans E.coli par transformation; aucun clone portant la résistance du plasmide n'a été obtenu) et que le transposon Tn5Sp^{r} était bien intégré dans le génome de SBL27Rif^{r} (par southern comme cela est décrit dans l'exemple 2).

Environ 3000 mutants de cette banque ont été réisolés sur milieu minimum M (1 g/l NH₄Cl, 7 g/l Na₂HPO₄, 3 g/l KH₂PO₄, 0,5 g/l NaCl, 1 mM MgSO₄, 0,1 mM CaCl₂, 10 mg/l thiamine) solide (15 g/l d'agar Difco) où la source de carbone est la glycine bétaïne à 10 g/l puis incubés à 30°C pendant 3 jours. Ensuite, la bétaïne, la diméthylglycine et la sarcosine ont été dosées dans le surnageant de culture après séparation par CLHP (colonne : Shodex Ionpak S 801 P, longueur 50 cm, diamètre 8 mm ; température: 70°C ; phase mobile : 0,01M azoture de sodium) et détection par réfractométrie différentielle.

Dix-huit mutants se sont révélés ne plus utiliser la bétaïne comme source de carbone, ce sont G3727 à G3731, G3733 à G3742 et G3897, G3899 et G3900.

D'autre part lorsque ces mutants sont cultivés, selon le protocole déjà décrit (Cameron et al, 1989), dans 10 ml de milieu PS4 pendant 5 jours à 30°C, de la bétaïne, ou de la diméthylglycine ou de la sarcosine peuvent être mesurées dans le surnageant de culture après séparation par CLHP et détection par réfractométrie différentielle. De la bétaïne est détectée dans le surnageant du moût des mutants G3728, G3736, G3897, G3899 et G3900 (entre 53 et 98 % de la bétaïne contenue dans le milieu initial) ; alors qu'avec les mutants G3727, G3738 et G3742 c'est de la diméthylglycine (environ 88 à 97 % de la bétaïne contenue dans le milieu initial) ; avec les mutants G3729, G3730, G3731, G3733, G3737 et G3741 c'est de la sarcosine (environ 64 à 75 % de la bétaïne introduite dans le milieu); avec les autres mutants G3734, G3735, G3739, G3740 et avec la souche non mutée aucun des trois produits n'est observé, voir tableau 1.

De plus, seuls SBL27 Rif^{r} et les mutants G3728, G3736, G3897, G3899 et G3900 poussent sur milieu minimum M solide où la source de carbone est la diméthylglycine (10 g/l). Des cultures en milieu minimum M, liquide, où la source de carbone est la glycine bétaïne à 10 g/l, sont réalisées comme suit : à partir d'un réisolement sur milieu riche LB (Cameron et al, 1989), la souche est cultivée dans 5 ml de milieu LB à 30°C pendant 12 heures ; les cellules de cette préculture sont lavées en milieu minimum M, puis des inoculums de 0,2 % sont ensemencés, dans huit Erlenmeyers de 250 ml contenant 25 ml de milieu minimum M où la source de carbone est la glycine bétaïne (10 g/l), et incubés sous agitation à 250 rpm, à 30°C. Aux jours 3, 4, 5, 6, 10, 14, 21, les cellules contenues dans un Erlenmeyer sont comptées après étalement et croissance sur milieu minimum solide où la source de carbone est la glycine bétaïne. Lorsque les mutants G3728 et G3900 sont cultivés en milieu minimum liquide où la source de carbone est la glycine bétaïne (10 g/l), pendant les trois premiers jours, aucune croissance n'est observée alors qu'avec la souche SBL27 Rif^{r} la phase stationnaire est déjà atteinte, voir figure 2 ; cependant au sixième jour une phase stationnaire comparable à celle de SBL27 Rif^{r} est observée. Ce résultat montre que les mutants qui ne dégradent pas toute la bétaïne (10 g/l) contenue dans le milieu PS4, voir tableau 1, sont capables de l'utiliser, mais après une phase de latence de 3 jours par rapport à la souche non mutée, comme source de carbone dans un milieu minimum contenant 10 g/l de bétaïne.

Ces données physiologiques permettent de mettre en évidence chez Pseudomonas denitrificans SBL27 Rif^{r} une voie de dégradation de la glycine bétaïne en diméthylglycine et sarcosine où :
1) l'étape de dégradation de la glycine bétaïne en diméthylglycine est bloquée (ou partiellement bloquée) chez les mutants G3728, G3736, G3897, G3899 et G3900 ;
2) l'étape de dégradation de la diméthylglycine en sarcosine est bloquée chez les mutants G3727, G3738 et G3742 ;
3) l'étape de dégradation de la sarcosine est bloquée chez les mutants G3729, G3730, G3731, G3733, G3737 et G3741.

### EXEMPLE 2 - Caractérisation génétique des mutants de Pseudomonas denitrificans bloqués dans le catabolisme de la bétaïne.

Cet exemple décrit les expériences de biologie moléculaire permettant de classer et caractériser les mutants à l'aide de leur transposon. Le génotype de chaque mutant est analysé par Southern (Maniatis et al, 1982) de la façon suivante : l'ADN génomique de chaque mutant (G3727 à G3731, G3733 à G3742 et G3897, G3899 et G3900) a été préparé puis une aliquote est digérée par l'enzyme de restriction EcoRI; les fragments ainsi obtenus sont séparés par électrophorèse sur un gel d'agarose puis sont transférés sur une membrane Biodyne; cette membrane est alors hybridée avec l'un des plasmides suivants marqué au α³²P-dCTP. Ces plasmides sont pT27, pT28, pT30, pT31, pT34, pT35, pT36, pT37, pT38, pT39, pT40, pT42, pT97, pT00. Ces plasmides ont été obtenus par insertion au site EcoRI du vecteur pRK290 de l'unique fragment EcoRI génomique dans lequel est inséré le transposon Tn5Sp^{r} des mutants G3727, G3728, G3730, G3731, G3734, G3735, G3736, G3737, G3738, G3739, G3740, G3742, G3897, G3900 respectivement. Les plasmides pT portant le fragment EcoRI recherché sont sélectionnés par leur résistance à la spectinomycine, conférée par le transposon. En hybridant la membrane par exemple avec le plasmide pT27, l'ADN génomique des mutants G3727 et G3738 ne présente qu'une seule bande radioactive correspondant à un fragment EcoRI de poids moléculaire d'environ 16 kb, ce qui indique que, dans ces mutants, le transposon est inséré au même endroit. En revanche, pour la souche non mutée, le fragment hybridant est de 8 kb et avec tous les autres mutants deux fragments hybrident, l'un comigre avec celui de la souche non mutée et l'autre possède une taille variable supérieure à la taille du transposon (8 kb) (de Bruijn, 1987 ; Prentki, 1984). Les hybridations successives avec chacun des plasmides ont permis de regrouper les mutants par classes d'hybridation de 1 à 12, voir tableau 1.

D'autre part les plasmides pT27, pT28, pT30, pT31, pT34, pT36, pT37, pT38, pT97, pT00 ont été utilisés pour réintroduire par double recombinaison homologue la mutation provenant de l'insertion du transposon, dans la souche non mutée SBL27 Rif^{r}, selon un protocole déjà décrit (Cameron et al, 1991). Pour les souches ainsi obtenues par disruption génique, le génotype a été vérifié par Southern comme cela vient d'être décrit dans le paragraphe précédent ; et le phénotype a été déterminé après analyse des composés détectés dans le surnageant des cultures en milieu PS4 comme décrit dans l'exemple 1.

Pour toutes les souches obtenues par disruption génique, qui conduisent au même phénotype que les souches mutées initiales, voir tableau 1, les quantités des composés détectés sont comparables. Ces souches appartiennent:
1) soit à la classe d'hybridation 5 et accumulent de la bétaïne,
2) soit à la classe d'hybridation 2 et accumulent de la diméthylglycine,
3) soit à la classe d'hybridation 4 et accumulent de la sarcosine.

De plus, une délétion d'un fragment génomique BglII de 10 kb et insertion d'une cassette de résistance à la spectinomycine ont été réalisées à partir du plasmide pT28. La souche, dont le génotype a été vérifié par Southern (comme cela est décrit dans le premier paragraphe de cet exemple) présente le même phénotype d'accumulation de bétaïne que les souches décrites dans la classe d'hybridation 5.

**Tableau 1. Classification des mutants bloqués dans le catabolisme de la bétaïne en sarcosine.**

| Souches n° G | Classes d'hybridation | Composés détectés chez les souches | |
|---|---|---|---|
| | | mutants initiaux | disruptants génétiques |
| 3727 | 2 | DMG 88 % | DMG |
| 3728 | 5 | Bétaïne 53 % sarcosine 13% | Bétaïne |
| 3729 | 3 | Sarcosine 68 % | ND |
| 3730 | 3 | Sarcosine 69 % | 0 |
| 3731 | 4 | Sarcosine 64 % | Sarcosine |
| 3733 | 4 | Sarcosine 69 % | ND |
| 3734 | 7 | 0 | 0 |
| 3735 | 8 | 0 | ND |
| 3736 | 1 | Bétaïne 62 % | 0 |
| 3737 | 4 | Sarcosine75% | Sarcosine |
| 3738 | 2 | DMG 91 % | DMG |
| 3739 | 9 | 0 | ND |
| 3740 | 10 | 0 | ND |
| 3741 | 4 | Sarcosine 64 % | ND |
| 3742 | 11 | DMG 97 % | ND |
| 3897 | 12 | Bétaïne 96 % | 0 |
| 3899 | 5 | Bétaïne 95 % | ND |
| 3900 | 5 | Bétaïne 98 % | Bétaïne |

### EXEMPLE 3 - Complémentation de mutants de Pseudomonas denitrificans bloqués dans les étapes de déméthylations de la glycine bétaine en sarcosine.

Cet exemple décrit l'isolement de fragments d'ADN de P.denitrificans portant des gènes impliqués dans la dégradation de la bétaïne en sarcosine. Ces fragments ont été mis en évidence par des expériences d'hybridation, en utilisant l'insert des plasmides pT28 ou pT38 comme sonde et la banque de plasmides contenant des fragments Sau3AI de l'ADN de P.denitrificans clonés dans pXL59 (Cameron et al, 1989). Les inserts des plasmides hybridant avec la sonde ont été cartographiés. Des clones ou des sous-clones, qui ont été construits (Maniatis et al, 1982) dans les vecteurs dérivés de RSF1010 (Cameron et al, 1989), ont été introduits par conjugaison (Cameron et al, 1989) chez les mutants bloqués dans le catabolisme de la bétaïne en diméthylglycine ou chez les mutants bloqués dans la dégradation de la diméthylglycine en sarcosine. La complémentation des mutants par les clones ou les sous-clones a été déterminée par l'absenoe d'accumulation de la bétaïne, diméthylglycine ou sarcosine dans le surnageant de culture des souches transconjuguants cultivées en milieu PS4, comme décrit dans l'exemple 1.

### 3-1 Fragment d'ADN de P.denitrificans portant des gènes impliqués dans la dégradation de la bétaïne en diméthylglycine.

Les clones hybridant avec pT28 ont été introduits chez les mutants G3728, G3736, G3897, G3899 et G3900 bloqués dans le catabolisme de la bétaïne en diméthylglycine. Comme cela est présenté sur la figure 3, deux sous-clones pXL2105 et pXL2107 contenus sur le fragment EcoRI de 12 kb complémentent quatre mutants parmi les cinq G3728, G3736, G3899 et G3900. L'un des sous-clones pXL2105 contient un fragment SstI-XhoI de 3,4 kb cloné dans le vecteur pXL435 (Cameron et al, 1989) et complémente deux mutants G3900 et G3899 ; l'autre pXL2107 contient un fragment BamHI-EcoRI de 4 kb cloné dans pKT230 (Cameron et al, 1989) et complémente les mutants G3728 et G3736. D'autre part l'insertion du transposon chez les mutants G3728, G3899 et G3900 a bien été cartographiée sur ce fragment EcoRI de 12 kb ; ce qui n'est pas observé chez le mutant G3736, il se peut que le phénotype de ce mutant ne soit pas corrélé à la position du transposon, puisque le mutant obtenu après génétique réverse n'accumule pas de bétaïne, voir l'exemple 2. Par conséquent au moins deux gènes sont impliqués dans l'étape de dégradation de la bétaïne en diméthylglycine.

### 3-2 Fragment d'ADN de P.denitrificans portant des gènes impliqués dans la dégradation de la diméthylglycine en sarcosine.

Les clones hybridant avec pT38 ont été introduits chez les mutants G3727, G3738 bloqués dans le catabolisme de la diméthylglycine en sarcosine. Comme cela est présenté sur la figure 4, deux clones issus de la banque et se cartographiant sur un fragment EcoRI-EcoRI-EcoRI de 14,5 kb complémente G3727 et G3738. L'un des sous-clones pXL19E2 contient un fragment EcoRI de 6,6 kb et l'autre pXL13C5 contient le fragment EcoRI-EcoRI-EcoRI de 14,5 kb. Par conséquent au moins un gène, cartographié sur le fragment décrit sur la figure 4, est impliqué dans l'étape de dégradation de la diméthylglycine en sarcosine.

### EXEMPLE b - Amélioration de la production de vitamine B₁₂ chez une souche Pseudomonas denitrificans modifiée dans le catabolisme de la glycine bétaïne.

Cet exemple illustre l'amélioration de la production de cobalamines d'une souche productrice de cobalamines par disruption de gènes impliqués dans le catabolisme de la bétaïne en diméthylglycine.

La mutation, qui est responsable du phénotype du mutant G3728 (ou G3900), est générée par le transposon qui est cartographié sur le fragment EcoRI, cloné dans le pT28 (ou pT00) et décrit dans l'exemple 2. Cette mutation a été introduite par double recombinaison homologue dans la souche de P.denitrificans SC510 Rif^{r}, productrice de cobalamines. (Cameron et al, 1991). Pour les deux souches ainsi obtenues SC510 Rif^{r}::pT28 et SC510 Rif^{r}::pT00 par génétique réverse, le génotype a été vérifié par Southern comme décrit dans l'exemple 2, et la production de cobalamines a été déterminée après analyse par CLHP (colonne : Asahibak OD50, longueur : 15 cm, diamètre : 6 mm, phase mobile : acétonitrile/ 0,1 M cyanure de potassium) et détection ultraviolette (longueur d'onde : 365 nm) de la vitamine B₁₂ contenue dans les cultures des souches poussées pendant sept jours à 30°C dans des Eclenmeyers de 250 ml contenant 25 ml de milieu PS4 où la glycine bétaïne est à 2 mg/l, selon un protocole déjà décrit (Cameron et al, 1989). Les résultats de ces dosages sont indiqués dans le tableau 2, ils représentent la valeur moyenne obtenue pour deux cultures dans les quatre expériences faites indépendemment.

Dans les conditions de culture utilisées, la production de cobalamines est augmentée d'un facteur 10, par rapport à la souche SC510Rif^{r}, chez les souches mutées dans un fragment génomique EcoRI de 13 kb, voir figure 3, dont l'ADN code pour des gènes impliqués dans le catabolisme de la bétaïne en diméthylglycine. Ces résultats montrent clairement qu'en modifiant le catabolisme de la bétaïne des souches de l'invention, leur capacité de production de métabolites a été augmentée.

**Tableau 2. Production de vitamine B₁₂ chez les souches mutées dans le fragment EcoRI portant des gènes impliqués dans le catabolisme de la bétaïne en diméthylglycine. Les niveaux de production des souches modifiées sont donnés par rapport à la production de la souche Sc510 Rif^{r}, qui a été arbitrairement fixée à 1.**

| Souches/Expérience | 1 | 2 | 3 | 4 |
|---|---|---|---|---|
| Sc510 Rif^{r} | 1 | 1 | 1 | 1 |
| SC510 Rif^{r}::pT28 | 10,5 | 11 | 22 | 14,5 |
| SC510 Rif^{r}::pT00 | 11 | 8 | | |

### Références bibliographiques

Berg D. et al., 1983, Bio/Technology 1 :417-435
F. de Bruijn. 1987. Methods in Enzymology.154 : 175-156.
B. Cameron, K. Briggs, S. Pridmore, G. Brefort and J. Crouzet. 1989. J. Bacteriol. 171:547-557.
B. Cameron, C. Guilhot, F. Blanche, L. Cauchois, M. Rouyez, S. Rigault, S. Levy-Schil and Crouzet. 1991. J. Bacteriol. 173:6058-6065.
S. Cayley, B. Lewis and T. Record. 1992. J. Bacteriol. 174:1586-1595.
L. Csonka. 1989. Microbiol. Rev. 53:121-147.
Ditta, G. et al., 1980, Proc.Natl.Acad.Sci. USA 77:7347-7351.
J. Graham and B. Wilkinson. 1992. J. Bacteriol. 174:2711-2716.
T. Maniatis, E. Fritsch and J. Sambrook. 1982. Molecular cloning: a laboratory manual. Cold Spring Harbor Laboratory, Cold Spring Harbor, New York.
A. Oren. 1990. Antonie van Leeuwenhoek 58:291-298.
P. Prentki and H. Krisch. 1984. Gene. 29:303-313.
Rothstein 1983, Meth. Enzymol. 101 202.
L Smith, J. Pocard, T. Bernard, D. Le Rudulier. 1988. J. Bacteriol. 170:3142-3149.
R. White and A. Demain. 1971. Biochim. Biophys. Acta. 237:112-119.

## Revendications

1. Procédé microbiologique de production de métabolites et/ou d'enzymes par culture d'une cellule productrice dudit métabolite et/ou enzyme dans des conditions de production puis récupération dudit métabolite et/ou enzyme, **caractérisé en ce que** la cellule productrice présente une modification au moins au niveau d'un gène impliqué dans le catabolisme de la bétaïne et **en ce que** la cellule dégrade moins rapidement la bétaïne.

2. Procédé selon la revendication 1, **caractérisé en ce** la ou les modifications sont des substitutions et/ou des insertions et/ou des délétions d'une ou plusieurs bases et/ou des disruptions.

3. Procédé selon la revendication 2, **caractérisé en ce que** la modification est stable ségrégationnellement et/ou non réversible.

4. Procédé selon les revendications 2 ou 3, **caractérisé en ce que** la ou les modifications portent sur la partie codante du ou desdits gènes et/ou sur les régions responsables de l'expression et/ou de la régulation transcriptionnelle desdits gènes.

5. Procédé selon les revendications 1 à 4, **caractérisé en ce que** la cellule est choisie parmi les cellules du genre Pseudomonas, Streptomyces, Actinomycetes, Propionibacterium, Corynebacterium, Bacillus, Escherichia, Salmonella, Rhizobium, Agrobacterium, Rhodopseudomonas, Xanthomonas, Clostridium et Methanobacterium.

6. Procédé selon les revendications 1 à 5, **caractérisé en ce que** le métabolite est choisi parmi les acides aminés, les vitamines, les antibiotiques, leurs dérivés ou leurs précurseurs.

7. Procédé selon la revendication 6, **caractérisé en ce que** le métabolite est une cobalamine, et de préférence la vitamine B12.

8. Cellule **caractérisée en ce qu'**il s'agit d'une cellule recombinée productrice de métabolites et/ou d'enzymes et **en ce qu'**elle présente une modification au moins au niveau d'un gène impliqué dans le catabolisme de la bétaïne, et **en ce que** la cellule dégrade moins rapidement la bétaïne.

9. Utilisation d'une cellule productrice de métabolites et/ou d'enzymes présentant une modification au moins au niveau d'un gène impliqué dans le catabolisme de la bétaïne, pour la production de métabolites et/ou enzymes, et en ce que la cellule dégrade moins rapidement la bétaïne.

## Patentansprüche

1. Mikrobiologisches Verfahren zur Produktion von Metaboliten und/oder Enzymen durch Kultur einer Produzentenzelle für den Metaboliten und/oder das Enzym unter Produktionsbedingungen, dann Gewinnung des Metaboliten und/oder des Enzyms, **dadurch gekennzeichnet, dass** die Produzentenzelle eine Modifikation wenigstens auf dem Niveau eines Gens, das beim Katabolismus von Betain beteiligt ist, aufweist und dass die Zelle das Betain weniger schnell abbaut.

2. Verfahren nach Anspruch 1, **dadurch gekennzeichnet, dass** die Modifikation(en), Substitutionen und/oder Insertionen und/oder Deletionen einer Base oder mehrerer Basen und/oder Durchtrennungen sind.

3. Verfahren nach Anspruch 2, **dadurch gekennzeichnet, dass** die Modifikation(en) stabil segregierend und/oder nichtreversibel ist.

4. Verfahren nach Anspruch 2 oder 3, **dadurch gekennzeichnet, dass** die Modifikation(en) auf dem codierenden Teil des Gens und der Gene und/oder auf den für die Expression und/oder die Transkriptionsregulation der Gene verantwortlichen Regionen getragen werden.

5. Verfahren nach den Ansprüchen 1 bis 4, **dadurch gekennzeichnet, dass** die Zelle unter den Zellen der Gattung Pseudomonas, Streptomyces, Actinomycetes, Propionibacterium, Corynebaceterium, Bacillus, Escherichia, Salmonella, Rhizobium, Agrobacterium, Rhodopseudomonas, Xanthomonas, Clostridium und Methanobacterium ausgewählt wird.

6. Verfahren nach den Ansprüchen 1 bis 5, **dadurch gekennzeichnet, dass** der Metabolit unter den Aminosäuren, den Vitaminen, den Antibiotika, ihren Derivaten oder ihren Vorläufern ausgewählt wird.

7. Verfahren nach Anspruch 6, **dadurch gekennzeichnet, dass** der Metabolit ein Cobalamin und vorzugsweise Vitamin B12 ist.

8. Zelle, **dadurch gekennzeichnet, dass** es sich um eine rekombinante Produzentenzelle für Metaboliten und/oder Enzyme handelt und dass sie eine Modifikation wenigstens auf dem Niveau eines Gens, das beim Katabolismus von Betain beteiligt ist, aufweist, und dass die Zelle Betain weniger schnell abbaut.

9. Verwendung einer Produzentenzelle für Metaboliten und/oder Enzyme, die eine Modifikation wenigstens auf dem Niveau eines Gens aufweist, welches beim Katabolismus des Betains beteiligt ist, für die Herstellung von Metaboliten und/oder Enzymen, wobei die Zelle das Betain weniger schnell abbaut.

## Claims

1. A microbiological process for producing metabolites and/or enzymes by culturing a cell that produces said metabolite and/or enzyme under production conditions, and then recovering said metabolite and/or enzyme, wherein the producing cell has a modification at least on a gene involved in the catabolism of betaine and wherein the cell degrades betaine less quickly.

2. The process as claimed in claim 1, wherein the modification(s) is (are) substitutions and/or insertions and/or deletions of one or more bases and/or disruptions.

3. The process as claimed in claim 2, wherein the modification is segregationally stable and/or irreversible.

4. The process as claimed in claim 2 or 3, wherein the modification(s) relate(s) to the coding part of said gene(s) and/or to the regions responsible for the expression and/or the transcriptional regulation of said genes.

5. The process as claimed in claims 1 to 4, wherein the cell is chosen from cells of the genera Pseudomonas, Streptomyces, Actinomycetes, Propionibacterium, Corynebacterium, Bacillus, Escherichia, Salmonella, Rhizobium, Agrobacterium, Rhodopseudomonas, Xanthomonas, Clostridium and Methanobacterium.

6. The process as claimed in claims 1 to 5, wherein the metabolite is chosen from amino acids, vitamins and antibiotics, derivatives thereof or precursors thereof.

7. The process as claimed in claim 6, wherein the metabolite is a cobalamin, and preferably vitamin B12.

8. A cell, wherein it is a recombinant cell that produces metabolites and/or enzymes and wherein it has a modification at least on a gene involved in the catabolism of betaine and wherein the cell degrades betaine less quickly.

9. The use of a cell that produces metabolites and/or enzymes, which has a modification at least on a gene involved in the catabolism of betaine, for the production of metabolites and/or enzymes, and wherein the cell degrades betaine less quickly.
